# EUROPEAN PATENT APPLICATION

(11) **EP 2 116 846 A1**
(43) Date of publication of application: **11.11.2009**
(21) Application number: 09150484.5
(22) Date of filing: 14.01.2009
(51) Int. Cl.: G01N 29/02, G01N 33/543, G01N 33/68

(54) **Surface acoustic wave immunosensor for diagnosing allergy disease and method for diagnosing allergy disease using the same**

(30) Priority: 08.05.2008 KR 20080042704
(71) Applicant: Samsung Electronics Co., Ltd., Suwon-si, Gyeonggi-do (KR)
(72) Inventor: Lee, Hun Joo, 449-712, Gyeonggi-do (KR); Jung, Sung Ouk, 449-712, Gyeonggi-do (KR); Shim, Jeo Young, 449-712, Gyeonggi-do (KR)
(74) Representative: Grünecker, Kinkeldey, Stockmair & Schwanhäusser Anwaltssozietät

(57) **Abstract**

A surface acoustic wave (SAW) immunosensor for diagnosing allergy disease includes one or more SAW devices on each of which allergens derived from one allergy-causing substance are fixed and an allergen derived from another allergy-causing substance is not included, that is, allergens derived from different allergy-causing substances being fixed on different SAW devices; and a signal detector which detects an output signal from the SAW device. Also a method for diagnosing allergy disease is capable of measuring levels of allergen-specific IgE and total IgE in blood or another sample taken from a subject of diagnosis using a SAW immunosensor, the allergens being derived from various allergy-causing substances, thereby capable of effectively diagnosing allergy disease for various allergy-causing substances.

## Description

### BACKGROUND

### 1. Field

Apparatuses and method consistent with the present invention relate to a surface acoustic wave (SAW) immunosensor capable of measuring levels of allergen-specific Immunoglobulin E (IgE) and total IgE in a sample such as blood taken from a subject of diagnosis using a surface acoustic wave, the allergens being derived from various allergy-causing substances, thereby capable of diagnosing allergy disease for various allergy-causing substances, and a method for diagnosing allergy disease using the same.

### 2. Description of the Related Art

When a signal is applied to an input electrode of a SAW device which uses a piezoelectric material, a SAW is generated. If a relevant substance binds to the surface of the SAW device, the surface mass of the SAW device changes and a difference in the SAW occurs from interaction between the substance and the SAW. At an output electrode, the SAW is converted into an electrical signal and outputted. By measuring the change of the output signal resulting from the substance, it is possible to detect the substance binding with the SAW device.

Allergy is one of hypersensitive reactions. Substances known as allergy-causing substances do not cause problems in most people. In some people, however, the substances may act as allergens and induce allergic diseases. Severe allergies may result in life-threatening anaphylactic reactions and potentially death. IgE is an antibody which is generated in blood in response to allergens or parasitic infections. Later, upon exposure to the same allergen, the allergen binds a Fab portion of the IgE and expels out the allergy-inducing substance. As a result, hypersensitive reactions such as asthma, atopy, food allergies, rhinitis, and the like occur.

For treatment or prevention of allergies, it is necessary to accurately and quickly detect an allergy-causing substance. Related art techniques for detecting an allergy-causing substance include skin prick testing, analyzing a serum for the presence and levels of allergen-specific IgE by chemiluminescence, utilizing an ELISA technique, and the like.

The above information disclosed in this Background section is only for enhancement of understanding of the background of the invention and therefore it may contain information that does not form a prior art that is already known in this country to a person of ordinary skill in the art.

### SUMMARY

The invention provides apparatuses and methods for effectively diagnosing allergy diseases for various allergy-causing substances by measuring levels of allergen-specific IgE and total IgE in blood or other sample using surface acoustic wave.

In an aspect, there is provided a SAW immunosensor for diagnosing allergy disease having: a surface acoustic wave (SAW) device on which one or more allergens derived from one allergy-causing substance (i.e., a single source of allergen(s)) are fixed and in which allergens derived from other allergy-causing substances are not included; and a signal detector which detects an output signal from the SAW device.

In another aspect, there is provided a method for diagnosing allergy disease using a SAW immunosensor having a SAW device. The method includes: an allergen reaction operation of reacting an allergen fixed on the SAW device with a sample taken from a subject of diagnosis; and a signal detection operation of detecting a signal outputted from the SAW device in response to the allergen reaction operation.

In still another aspect, there is provided a method for diagnosing allergy disease using a SAW immunosensor having a SAW device. The method includes: an allergen fixing operation of fixing an allergen derived from an allergy-causing substance on a SAW device; an allergen reaction operation of reacting the allergen fixed on the SAW device with a sample taken from a subject of diagnosis; and a signal detection operation of detecting a signal outputted from the SAW device in response to the allergen reaction operation.

### BRIEF DESCRIPTION OF THE DRAWINGS

The above and other aspects of the present invention will be more clearly understood from the following detailed description taken in conjunction with the accompanying drawings, in which:

FIG. 1 schematically shows an exemplary embodiment for detecting allergen-specific IgE using a surface acoustic wave (SAW) immunosensor comprising a SAW device on which allergens derived from one allergy-causing substance are fixed;

FIG. 2 schematically shows an exemplary embodiment for detecting total IgE using a SAW immunosensor comprising a SAW device on which receptors that specifically bind with IgE Fc are fixed;

FIG. 3 shows a result of analyzing ragweed-specific IgE according to an exemplary embodiment. Frequency signals of a test SAW device and a reference SAW device are graphically shown; and

FIG. 4 shows a result of analyzing ragweed-specific IgE according to an exemplary embodiment. Differences in frequency signals of a test SAW device and a reference SAW device are graphically shown.

### DETAILED DESCRIPTION

Hereinafter, reference will be made in detail to various embodiments, examples of which are illustrated in the accompanying drawings and described below. While the invention will be described in conjunction with exemplary embodiments, it will be understood that the present description is not intended to limit the invention to those exemplary embodiments. On the contrary, the invention is intended to cover not only the exemplary embodiments, but also various alternatives, modifications, equivalents and other embodiments, which may be included within the spirit and scope of the invention as defined in the appended claims.

A surface acoustic wave (SAW) immunosensor may comprise a signal generator, a SAW device which generates a SAW based on the signal generated by the signal generator and outputs the SAW after it has traveled a predetermined distance as an output signal, and a signal detector which detects the output signal from the SAW device, and thereby, senses a substance that binds with the SAW device.

In an exemplary embodiment, an output signal from the SAW immunosensor may be applied again as an input signal for generating a SAW in the SAW immunosensor (oscillation). In case a plurality of SAW immunosensors are used, each sensor may be oscillated independently. Alternatively, a network analyzer may be used to generate a signal with a predetermined frequency outside a SAW immunosensor and apply it to the SAW immunosensor.

In an exemplary embodiment, a SAW device may comprise one or more interdigital transducer (IDT) for converting an applied electrical signal to a SAW, which is a mechanical wave, and converting the SAW again to an electrical signal for output.

In an exemplary embodiment, a biomaterial such as IgE may be detected using a SAW device by fixing a receptor, that specifically binds with the biomaterial to be detected, on the SAW device, and measuring a change of an output signal resulting from a weight change accompanied by the binding of the biomaterial with the receptor. As such, a qualitative analysis is possible from presence or absence of the biomaterial bound to the receptor, and a quantitative analysis is also possible from the change of the output signal.

In an exemplary embodiment, one or more allergens derived from one allergy-causing substance are fixed on one or more SAW devices. Each of the SAW devices should not include an allergen derived from other allergy-causing substance(s). A sample taken from a subject of diagnosis is provided on the one or more SAW devices, so that an IgE included in the sample reacts with the allergen(s) fixed on the SAW devices. Then, a change of mass of each SAW device in response to binding with the IgE may be measured as a change of a SAW. When a change of a SAW signal is measured for a particular SAW device, it implies that the subject of diagnosis has IgE specific to the allergen fixed on the corresponding SAW device. Accordingly, it can be diagnosed that the subject of diagnosis is allergic to the allergy-causing substance from which the allergen is derived. Also, since the mass change based on the level of IgE bound specifically to the allergen fixed on the SAW device varies depending on concentration of IgE in the sample, it is possible to quantitatively analyze the IgE level in the sample.

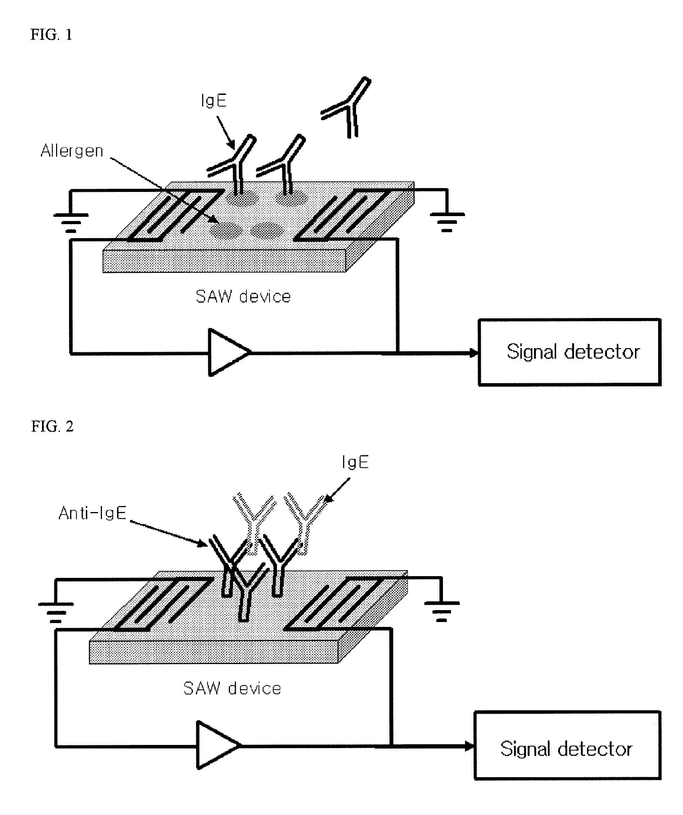

FIG. 1 schematically shows an exemplary embodiment for detecting allergen-specific IgE using a SAW immunosensor comprising a SAW device on which allergens derived from an allergy-causing substance are fixed. When IgE binds with any of the allergen(s), a change in frequency, phase, amplitude or clock number of SAW can be measured. The signal detector for detecting a signal change may be a frequency counter, a phase detector, a network analyzer, an oscilloscope, or the like. In an exemplary embodiment, the change in frequency is measured.

In another exemplary embodiment, the SAW immunosensor may further comprise a SAW device on which receptors that can bind with IgE Fc are fixed for measurement of total IgE in a sample taken from a subject of diagnosis. From the measurement of total IgE, it is possible to diagnose a risk of overall allergy diseases and other immune-related diseases of the subject of diagnosis. The receptors may be antibodies that specifically bind only with IgE Fc (IgG, IgA, IgM, etc.), aptamers, or FcεRI receptor expressed on mast cells, which specifically bind with mast cells and IgE Fc. FIG. 2 schematically shows an exemplary embodiment for detecting total IgE using a SAW immunosensor comprising a SAW device on which receptor that specifically bind with IgE Fc are fixed.

In an exemplary embodiment, the allergy-causing substance may be any substance that may induce allergies in a human body, including food, pollen, tick, mold, grass, animal fur, metal, plastic, rubber, medicine, and the like. Each of the allergy-causing substances includes one or more allergens. For example, the allergy-causing *Aspergillus fumigatus* includes such allergens as peroxysomal proteins, thioredoxins, metalloproteases, and the like. The allergen may be either a natural allergen extracted from an allergy-causing substance or one prepared using a recombinant protein.

In an exemplary embodiment, the SAW immunosensor may further comprise a reference SAW device on which any allergen is not fixed, for comparison of a signal change with the SAW device on which an allergen is fixed (test SAW device). The SAW immunosensor may further comprise a comparator unit for the comparison. In this case, the change of a SAW signal resulting from binding of IgE on the test SAW device can be detected with higher sensitivity, because a noise resulting from variation in density, viscosity, temperature and pressure of the sample other than mass can be offset. The number of the test SAW devices can be determined depending on the number of allergy-causing substances needed to be screened.

In an exemplary embodiment, the one or more test SAW devices and the reference SAW device may be disposed in a single chamber or in different chambers. In case they are disposed in a single chamber, a sample taken from a subject of diagnosis can be injected to the chamber at once. And, in case they are disposed in different chambers, the sample taken from a subject of diagnosis can be injected to each of the chambers. In an exemplary embodiment, the sample taken from a subject of diagnosis may be whole blood, serum or plasma. The sample may be injected onto the SAW immunosensor using a pump. As used herein, plasma refers to a liquid component of blood, excluding red blood cells, white blood cells, platelets, and the like. And, serum refers to a remainder of plasma excluding fibrinogens.

In an exemplary embodiment, the SAW device may be either a transverse type or a resonator type. A transverse type gives a broad peak, whereas a resonator type gives a sharp peak. But, either of the two types may be used.

In an exemplary embodiment, the SAW immunosensor may generate a SAW by applying an output signal from the SAW device again to the SAW device as an input signal (oscillation).

The exemplary SAW immunosensor and the diagnosis method can prevent a pain and a risk of anaphylaxis caused by hypersensitive reactions associated with the skin prick testing in which allergens are directly introduced into the skin of a subject using a needle. Further, allergy testing is possible for babies or infants to whom the skin prick testing is inadequate. Because the exemplary SAW immunosensor and the diagnosis method enable a direct testing, enzymes, fluorescent reagents or other expensive reagents required in chemiluminescence are not needed. Further, the testing time can be reduced because such procedures as secondary antibody binding, staining, washing, and the like can be eliminated. In particular, an exact and sensitive allergy diagnosis for allergy-causing substances including various allergens may be possible.

The following example is directed to detection of ragweed-specific IgE to illustrate the invention, but is not intended to limit the same.

Ragweed is a flowering plant from the sunflower family. Its pollen is known to cause severe and widespread allergies.

In order to detect ragweed-specific IgE, ragweed was fixed on a surface of a test SAW device according to an exemplary embodiment of the present invention using 3-aminopropyltriethoxysilane (APTES) and glutaraldehyde. On a surface of a reference SAW device, BSA (bovine serum albumin) was fixed using APTES and glutaraldehyde. The allergen fixed test SAW device and the reference SAW device were disposed in a chamber and mounted respectively on an oscillator. After stabilizing for 1 minute by injecting PBS buffer in the chamber, a sample including ragweed-specific IgE at a concentration of 15 kU/L was injected for 30 seconds. Then, after monitoring a reaction in real time for 3 minutes and 30 seconds, the sample was washed with PBS for 1 minute. A qualitative analysis on the presence of ragweed-specific IgE and a quantitative analysis of IgE were carried out by comparing a signal difference of the test SAW device and the reference SAW device.

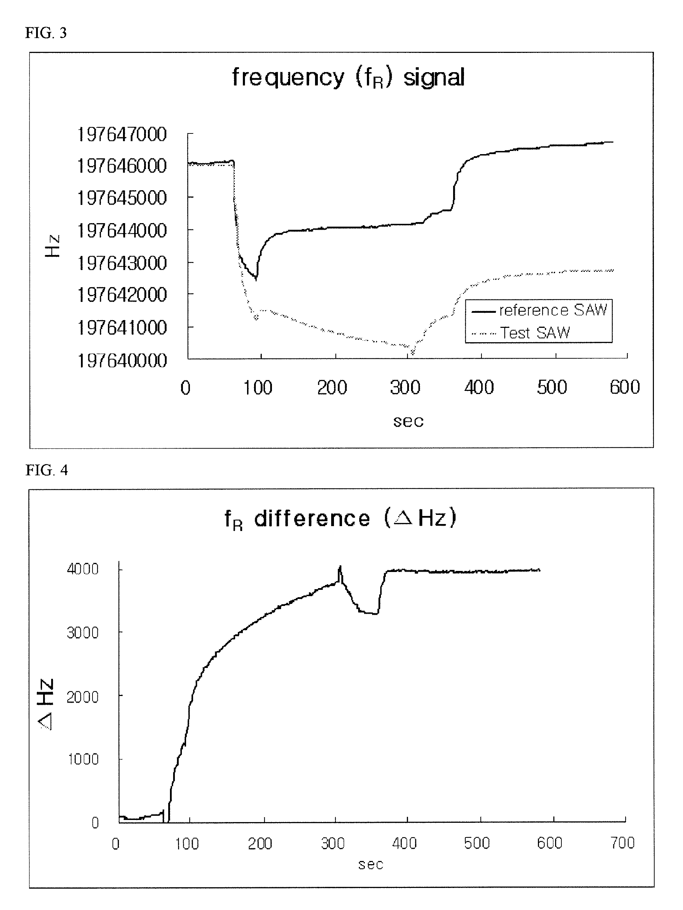

The analysis result is shown in FIG. 3. As seen in FIG. 3, the two SAW frequency signals which remained stable for 1 minute dropped because of a change in pressure and viscosity when the sample was injected. When the sample injection was stopped 30 seconds later, the signal of the test SAW device dropped further as the reaction with the IgE included in the sample continued, while the signal of the reference SAW device remained constant. After the sample was washed with PBS, all the reaction stopped and both signals of the test SAW device and the reference SAW device remained constant. FIG. 4 is a graph obtained by calculating a difference of the two SAW signals. From a frequency change and a rate of change seen in FIG. 4, the ragweed-specific IgE included in the sample can be analyzed quantitatively.

Although the exemplary embodiments of the present invention have been disclosed for illustrative purposes, those skilled in the art will appreciate that various modifications, additions and substitutions are possible, without departing from the scope of the invention as disclosed in the accompanying claims.

## Claims

1. A surface acoustic wave (SAW) immunosensor for diagnosing allergy disease comprising
at least one test SAW device on which one or more allergens derived from one allergy-causing substance are fixed, and in which an allergen derived from other allergy-causing substance is not included; and
a signal detector which detects an output signal from the test SAW device.

2. The SAW immunosensor for diagnosing allergy disease according to claim 1, further comprising a reference SAW device on which no allergen is fixed.

3. The SAW immunosensor for diagnosing allergy disease according to claim 2, further comprising a comparator which compares the output signal from the test SAW device with an output signal from the reference SAW device.

4. The SAW immunosensor for diagnosing allergy disease according to any one of claims 1 to 3, wherein the at least one test SAW device comprises a plurality of test SAW devices including the first test SAW device, and
wherein allergens derived from different allergy-causing substances are fixed on the plurality of test SAW devices.

5. The SAW immunosensor for diagnosing allergy disease according to any one of claims 1 to 4, further comprising a SAW device on which a receptor that specifically binds with IgE Fc is fixed.

6. The SAW immunosensor for diagnosing allergy disease according to claim 5, wherein the receptor is at least one selected from a group consisting of IgG that specifically binds with IgE Fc, IgA that specifically binds with IgE Fc, IgM that specifically binds with IgE Fc, an aptamer, an FcεRI receptor, expressed on mast cells, which specifically binds with the mast cells and IgE Fc, and any mixtures thereof

7. The SAW immunosensor for diagnosing allergy disease according to any one of claims 1 to 6, wherein the signal detector detects at least one of frequency, phase, amplitude and clock number of the output signal from at least one of the test SAW devices.

8. A method for diagnosing allergy disease using a surface acoustic wave (SAW) immunosensor comprising a test SAW device, the method comprising:
reacting an allergen fixed on the test SAW device with a sample taken from a subject of diagnosis; and
detecting a signal outputted from the test SAW device in response to the reacting.

9. A method for diagnosing allergy disease, the method comprising:
fixing an allergen derived from an allergy-causing substance on a first test surface acoustic wave (SAW) device;
reacting the allergen fixed on the first test SAW device with a sample taken from a subject of diagnosis; and
detecting a signal outputted from the first test SAW device in response to the reacting.

10. The method for diagnosing allergy disease according to claim 8 or 9, further comprising:
detecting a reference signal outputted from a reference SAW device on which no allergen is fixed; and
comparing the detected signal outputted from the test SAW device with the reference signal.

11. The method for diagnosing allergy disease according to any one of claims 8 to 10, wherein the allergen comprises a receptor that specifically binds with IgE Fc, and
wherein the detecting the signal outputted from the test SAW device in response to the reacting comprises measuring a content of total IgE in the sample.

12. The method for diagnosing allergy disease according to claim 11, wherein the receptor is at least one selected from a group consisting of IgG that specifically binds with IgE Fc, IgA that specifically binds with IgE Fc, IgM that specifically binds with IgE Fc, an aptamer, an FcεRI receptor, expressed on mast cells, which specifically binds with the mast cells and IgE Fc, and any mixtures thereof

13. The method for diagnosing allergy disease according to any one of claims 9 to 12, wherein the method uses a plurality of test SAW devices, including the first test SAW device, on which allergens derived from different allergy-causing substances are fixed.

14. The method for diagnosing allergy disease according to any one of claims 8 to 13, wherein the sample taken from the subject of diagnosis is at least one selected from whole blood, serum and plasma.

15. The method for diagnosing allergy disease according to any one of claims 8 to 14, wherein, in the detecting the signal, at least one of frequency, phase, amplitude and clock number of the output signal from the test SAW device is detected.
